(19) Europäisches Patentamt
European Patent Office
Office européen des brevets

(11) Veröffentlichungsnummer: **0 008 108**
**B1**

# (12) EUROPÄISCHE PATENTSCHRIFT

(45) Veröffentlichungstag der Patentschrift: **05.05.82**

(51) Int. Cl.³: **C 07 D 207/333**, C 07 D 405/12, A 61 K 31/40

(21) Anmeldenummer: **79102837.6**

(22) Anmeldetag: **07.08.79**

(54) **Aminopropanolderivate des 2-(o-Hydroxyphenyl)-pyrrols, Verfahren zu ihrer Herstellung und diese enthaltende pharmazeutische Zubereitungen.**

(30) Priorität: **12.08.78 DE 2835438**

(43) Veröffentlichungstag der Anmeldung: **20.02.80 Patentblatt 80/4**

(45) Bekanntmachung des Hinweises auf die Patenterteilung: **05.05.82 Patentblatt 82/18**

(84) Benannte Vertragsstaaten: **AT BE CH DE FR GB IT LU NL SE**

(56) Entgegenhaltungen:
**CH-A-575 922**
**CH-A-575 923**
**CH-A-575 924**
**CH-A-579 045**
**CH-A-579 046**

(73) Patentinhaber: **BASF Aktiengesellschaft, Carl-Bosch-Strasse 38, D-6700 Ludwigshafen (DE)**

(72) Erfinder: **Frickel, Fritz-Frieder, Dr., Chem., Prager Strasse 27, D-6700 Ludwigshafen (DE)**
Erfinder: **Franke, Albrecht, Dr., Chem., Mandelring 11, D-6706 Wachenheim (DE)**
Erfinder: **Koenig, Horst, Dr., Chem., Pariser Strasse 4, D-6700 Ludwigshafen (DE)**
Erfinder: **Lenke, Hans-Dieter, Dr., Kekulé-Platz 1, D-6700 Ludwigshafen (DE)**
Erfinder: **Gries, Josef, Dr., Roemerweg 43, D-6706 Wachenheim (DE)**

**EP 0 008 108 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Aminopropanolderivate des 2-(o-Hydroxyphenyl)-pyrrols, Verfahren zu ihrer Herstellung und diese enthaltende pharmazeutische Zubereitungen

Die Erfindung betrifft Aminopropanolderivate des 2-(o-Hydroxyphenyl)-pyrrols und ihre Säureadditionssalze, ihre Herstellung und diese Verbindungen enthaltende pharmazeutische Zubereitungen, die bei der Behandlung von Herz- und Kreislauferkrankungen verwendet werden können.

In der CH-A 575 923 werden Aminopropanolderivate des o-(1-Pyrrolyl)-phenols, Verbindungen, in denen der Pyrrolring über das Ringstickstoffatom mit dem Phenol verbunden ist, beispielsweise das 1-tert.-Butylamino-3-[o-(pyrrol-1-yl)-phenoxy]-2-propanol, beschrieben. Für diese Verbindungen wird eine β-sympatholytische Wirkung, die sie zur Behandlung von Herz- und Kreislauferkrankungen geeignet erscheinen lassen, angegeben. Die gefundene Wirkung befriedigt jedoch nicht immer.

Es wurde nun gefunden, dass Verbindungen der allgemeinen Formel (I)

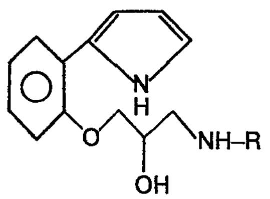

(I)

in der R einen am zum Stickstoffatom α-ständigen C-Atom verzweigten Alkylrest mit 3 bis 6 C-Atomen bedeutet und ihre physiologisch verträglichen Säureadditionssalze wertvolle pharmakologische Eigenschaften aufweisen.

Alkylreste für R sind beispielsweise Isopropyl, tert.-Butyl, 2-Methyl-butyl-2, Butyl-2, 3-Methylpentyl-3 und Pentyl-2.

Neben den in den Beispielen angegebenen Verbindungen können als erfindungsgemässe Verbindungen beispielsweise genannt werden:

2-[2-[3-(2-Methyl-butyl-2-amino)-2-hydroxy-propoxy]-phenyl]-pyrrol

2-[2-[3-(3-Methyl-pentyl-3-amino)-2-hydroxy-propoxy]-phenyl]-pyrrol

2-[2-[3-(2,3-Dimethyl-butyl-2-amino)-2-hydroxy-propoxy]-phenyl]-pyrrol

Die erfindungsgemässen Verbindungen werden hergestellt, indem man ein arylsubstituiertes Pyrrol der allgemeinen Formel (II)

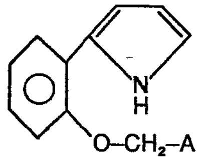

(II),

in der A den Rest 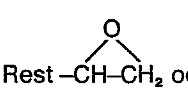 oder 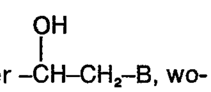, wobei B für eine nukleofuge Abgangsgruppe steht, bedeutet, mit einem Amin der allgemeinen Formel

$$H_2N–R,$$

in der R die oben angegebenen Bedeutungen hat, zweckmässigerweise in einem Lösungsmittel und gegebenenfalls in Gegenwart eines säurebindenden Mittels in an sich bekannter Weise umsetzt und die erhaltene Verbindung gegebenenfalls in ein physiologisch verträgliches Säureadditionssalz überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäureester in Betracht, wie der p-Toluolsulfonsäure-, der p-Brombenzolsulfonsäure- oder der Methansulfonsäurerest.

Die Umsetzungen werden bei Raumtemperatur oder bei höheren Temperaturen, zweckmässig bei Temperaturen von 50 bis 120°C, durchgeführt. Die Umsetzungen können unter Atmosphärendruck oder in einem geschlossenen Gefäss unter erhöhtem Druck gegebenenfalls unter Erwärmung auf den angegebenen Temperaturbereichen durchgeführt werden.

Die Ausgangsverbindungen können direkt, d.h. ohne Zugabe eines Verdünnungs- oder Lösungsmittels, umgesetzt werden. Zweckmässigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, von Benzol oder eines Alkylbenzols, wie Toluol oder Xylol, oder eines aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Auch ist das in überschüssiger Menge verwendete Amin der Formel $H_2N–R$ gegebenenfalls als Verdünnungs- oder Lösungsmittel geeignet.

Bevorzugte Lösungsmittel bei der Umsetzung von 2-[2-(2,3-Epoxypropoxy)-phenyl]-pyrrol mit einem Amin $R–NH_2$ sind niedere Alkohole, insbesondere Äthanol oder Isopropanol, wobei die Umsetzung bevorzugt bei Temperaturen von 50 bis 100°C und bei Normaldruck durchgeführt wird. Bei der nukleophilen Substitution eines Restes B sind als Lösungsmittel ein niederes aliphatisches Keton, wie Aceton, Methyläthylketon oder Methylisobutylketon, ein cyclischer Äther, insbesondere Tetrahydrofuran oder Dioxan, oder ein Dialkylformamid, wie Dimethylformamid, und Temperaturen von 90 bis 120°C zweckmässig. Gegebenenfalls wird die Reaktion in Gegenwart einer katalytischen Menge Natrium-oder Kaliumjodid durchgeführt.

Es sei erwähnt, dass als Ausgangsverbindung

der Formel (II) gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmässigen Ausführungsform zur nukleophilen Substitution des Restes B durch das verwendete Amin wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuss verwendet. Gegebenenfalls ist es zweckmässig, das zur Umsetzung verwendete Amin $H_2N–R$ in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung des 2-(o-Hydroxyphenyl)-pyrrols mit einem Epihalogenhydrin oder einem α,ω-Dihalogen-2-propanol erhalten werden. Als Epihalogenhydrine kommen Epichlorhydrin, Epibromhydrin und Epijodhydrin und als α,ω-Dihalogen-2-propanol kommen insbesondere 1,3-Dichlor-2-propanol und 1,3-Dibrom-2-propanol in Betracht.

Die Umsetzungen des 2-(o-Hydroxyphenyl)-pyrrols zur Herstellung der Ausgangsverbindungen der Formel (II) werden zweckmässigerweise bei Temperaturen von 0 bis 120°C und unter Normaldruck oder in einem geschlossenen Gefäss unter erhöhten Drucken durchgeführt. Die Umsetzungen werden zweckmässigerweise in einem inerten Verdünnungs- oder Lösungsmittel, z. B. einem niederen aliphatischen Keton, wie Aceton, Methyläthylketon oder Methylisobutylketon, einem niederen Alkohol mit 1 bis 4 C-Atomen, wie Methanol, Äthanol, Propanol oder Butanol, einem aliphatischen oder cyclischen Äther, wie Dialkyläther, Tetrahydrofuran oder Dioxan, einem Dialkylformamid, wie Dimethylformamid oder Diäthylformamid, oder Dimethylsulfoxid oder Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuss verwendet werden.

Bevorzugt wird das 2-(o-Hydroxyphenyl)-pyrrol mit Epibromhydrin oder 1,2-Dibrompropanol-2 in einem Lösungsmittelgemisch aus einem Äther und einem polaren aprotischen Lösungsmittel, insbesondere Tetrahydrofuran und Hexamethylphosphortriamid, oder einem Keton, wie Aceton, bei Temperaturen zwischen 0 und 50°C umgesetzt.

Die erfindungsgemässen Verbindungen der Formel (I) besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Brom-cam-pher-8-sulfonsäure, in die optisch aktiven Antipoden getrennt werden können.

Gegebenenfalls werden die erhaltenen erfindungsgemässen Verbindungen in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure, und als organische Säuren beispielsweise Oxalsäure, Maliensäure, Fumarsäure, Milchsäure, Weinsäure, Apfelsäure, Zitronensäure, Salicylsäure, Adipinsäure oder Benzoesäure oder können aus «Fortschritte der Arzneimittelforschung», Band 10, Seiten 224 bis 225, Birkhäuser Verlag, Basel und Stuttgart, 1966, oder dem «Journal of Pharmaceuticals Sciences», Volume 66, Seiten 1 bis 5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wässrige Lösungen von Säure-Additionsverbindungen der Aminopropanol-Derivate der allgemeinen Formel (I) durch Auflösen der freien Basen der allgemeinen Formel (I) in einer wässrigen Säurelösung hergestellt werden.

Die erfindungsgemässen Verbindungen und ihre physiologisch verträglichen Säureadditionssalze weisen wertvolle pharmakologische Eigenschaften auf und können bei Herz- und Kreislauferkrankungen verwendet werden. Sie sind aufgrund ihrer β-sympatholytischen Wirkung zur Behandlung der coronaren Herzkrankheit, der Hypertonie und von Herzrhythmusstörungen geeignet. Im Hinblick auf die Verwendung, Wirkung und Bestimmung von β-Blockern sei beispielsweise auf die Literaturstelle C.T. Dollery et al, «Clinical Pharmacology and Therapeutics», Band 10, Nr. 6, 765–799, 1969, und den dort genannten Literaturstellen verwiesen.

Die β-sympatholytische Wirkung wurde im Vergleich mit dem bekannten β-Sympatholytikum Propanolol (1-(Isopropylamino)-1-naphthyloxy-2-propanol · HCl) an Ratten getestet. Dazu wurden folgende Methoden benutzt:

1. $\beta_1$-sympatholytische Wirkung

Isoproterenol (0,1 ug/kg intravenös) verursacht an despinalisierten Ratten (Stamm: Sprague-Dawley, Mus Rattus; Gewicht 230 bis 280 g) Herzfrequenzsteigerungen um durchschnittlich 45%. β-Sympatholytica hemmen diese Tachycardien. Isoproterenol wurde vor und 5 min nach der intravenösen Applikation der Prüfsubstanzen appliziert. Zwischen den Logarithmen der applizierten Dosen (mg/kg) der Prüfsubstanzen und der Hemmung der Isoproterenol-Tachycardie (%) bestehen lineare Beziehungen. Aus diesen Beziehungen werden als ED 50% die Dosen ermittelt, welche die Isoproterenol-Tachycardie um 50% hemmen.

2. Akute Toxizität

Die akute Toxizität wurde an Gruppen von je 10 weiblichen NMRI-Mäusen, Gewicht 19 bis 27 g, bei intraperitonealer Applikation ermittelt. Als LD 50 wurde die Dosis berechnet (Probit-Analyse), nach der 50% der Tiere innerhalb von 24 Stunden starben.

Die Tabelle 1 zeigt, dass die pharmakotherapeutisch wichtige $\beta_1$-sympatholytische Aktivität der erfindungsgemässen Verbindungen 5,6- (Beispiel 1) bzw. 3,2mal (Beispiel 2) stärker ist als die der Vergleichssubstanz Propranolol. Die therapeutische Breite als Quotient aus der letalen Dosis (LD 50) und der $\beta_1$-blockierenden Dosis (ED 50%) ist 4- (Beispiel 2) bzw. 7mal (Beispiel 1) grösser als die von Propranolol.

Tabelle 1

| Verbindung | $\beta_1$-sympatholytische Wirkung[1] ED 50%[2] | R.W.[3] | Akute Tox. LD 50[4] | Therapeutische Breite[5] absolut | relativ[6] |
|---|---|---|---|---|---|
| Propranolol | 0,0127 | 1,00 | 108 | 111 | 1,00 |
| Beispiel 1 | 0,00227 | 5,59 | 165 | 767 | 6,91 |
| Beispiel 2 | 0,004 | 3,18 | 97,2 | 452 | 4,07 |

[1] Hemmung der Isoproterenol-Tachycardie. Ratte despinalisiert. Appl.: i.V.
[2] Dosis, welche die Isoproterenol-Tachycardie um 50% hemmt.
[3] Relative Wirksamkeit. Propranolol = 1,00.
[4] Maus. Appl.: i.p.
[5] $\frac{\text{LD 50}}{\text{ED 50\%}}$
[6] Propranolol = 1,00.

Gegenstand der vorliegenden Erfindung sind demnach auch therapeutische Mittel oder Zubereitungen, die neben üblichen Träger- und Verdünnungsmitteln eine Verbindung der Formel (I) als Wirkstoff enthalten, sowie die Verwendung der neuen Verbindungen bei der Behandlung von Herz- und Kreislauferkrankungen.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in an sich bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Als Einzeldosis der erfindungsgemässen Verbindungen kommen am Menschen 1 bis 100 mg, vorzugsweise 2 bis 50 mg, in Betracht.

Bezüglich ihrer Wirksamkeit sind besonders hervorzuheben das
2-[2-(3-tert.-Butylamino-2-hydroxy-propoxy)phenyl]-pyrrol und das
2-[2-(3-Isopropylamino-2-hydroxy-propoxy)-phenyl]-pyrrol.

Die Erfindung wird durch die nachfolgenden Ausführungsbeispiele näher erläutert.

I. Herstellung von Ausgangsverbindungen

1. o-Benzyloxybenzoesäureallylamid

A. 30 g o-Benzyloxybenzoesäuremethylester und 15 ml Allylamin werden 7 Tage unter Rückfluss gekocht. Der Rückstand wird aus Äther bei −25°C umkristallisiert.

Ausbeute: 17,2 g, was 52% der Theorie entspricht; Schmp. 48°C.

B. 4,9 g (20 mMol) O-Benzylsalicylsäurechlorid in 10 ml Dioxan werden langsam in eine eisgekühlte Lösung von 1,7 g (30 mMol) Allylamin und 4 g Natriumhydrogencarbonat in 100 ml Wasser eingetragen und über Nacht gerührt. Das Produkt wird abgesaugt und wie unter A. umkristallisiert.

Ausbeute: 4,3 g, was 80% der Theorie entspricht.

2. 2-(o-Benzyloxyphenyl)-pyrrol

2,7 g (10 mMol) o-Benzyloxybenzoesäureallylamid werden in 20 ml einer 20gewichtsprozentigen Lösung von Phosgen in Toluol unter Zusatz von 2 Tropfen Dimethylformamid über Nacht bei Raumtemperatur gerührt. Der nach dem Abdestillieren des Toluols unter vermindertem Druck bei 40°C Badtemperatur verbleibende Rückstand wird in 30 ml Tetrahydrofuran aufgenommen, über Glaswatte filtriert und innerhalb von 45 Minuten in eine eisgekühlte Lösung von 4,0 g (30 mMol) Kalium-tert.-butylat in 60 ml Tetrahydrofuran/Benzol (1 : 1 Vol.) unter Argon als Schutzgas eingetragen. Zehn Minuten nach beendeter Zugabe wird das Lösungsmittel unter vermindertem Druck abdestilliert, man verteilt den Rückstand zwischen Methylenchlorid und Wasser und extrahiert zweimal mit Methylenchlorid.

Die über Natriumsulfat getrockneten, vereinigten organischen Phasen werden eingedampft und der Rückstand über eine kurze Kieselgelsäure (30 × 2 cm) mit Tetrachlorkohlenstoff als Eluationsmittel chromatographiert. Das nach dem Einengen der Eluate verbleibende Öl wird aus Methanol umkristallisiert.

Ausbeute: 1,4 g, das entspricht 56% der Theorie; Schmp. 72°C.

Analyse: $C_{17}H_{15}NO$ 249,3

| | | | |
|---|---|---|---|
| ber.: | 81,90 C | 6,06 H | 5,62 N |
| gef.: | 81,76 C | 6,00 H | 5,45 N |

3. 2-(o-Hydroxyphenyl)-pyrrol

Zu 1,5 g (6 mMol) 2-(o-Benzyloxyphenyl)pyrrol in 30 ml Methanol gibt man 150 mg 10%igen Palladium-Kohle-Katalysator und hydriert unter leichtem Überdruck. Der nach dem Abtrennen des Katalysators und Eindampfen verbleibende Rückstand wird aus Toluol/Petroläther (40/60°C) umkristallisiert. Die Ausbeute von 0,8 g entspricht 84% der Theorie, Schmp. 100 bis 101°C.

Analyse: $C_{10}H_9NO$ 159,19

| | | | |
|---|---|---|---|
| ber.: | 75,45 C | 5,70 H | 8,80 N |
| gef.: | 75,40 C | 5,70 H | 8,76 N |

4. 2-[2-(2,3-Epoxypropoxy)-phenyl]-pyrrol

7,0 g 2-(o-Hydroxyphenyl)-pyrrol, 7,3 g Epibromhydrin und 11,4 g trockenes Kaliumcarbonat werden in 50 ml Aceton 7 Stunden auf Rückflusstemperatur erhitzt. Nach dem Erkalten wird abfiltriert und der Filterrückstand mit Aceton gewaschen. Die vereinigten Filtrate werden durch Abdestillieren vom Lösungsmittel befreit. Der zweimal an Kieselgel mit Methylenchlorid als Elutionsmittel chromatographierte Rückstand ergibt 4,6 g (48% der Theorie) 2-[2-(2,3-Epoxypropoxy)-phenyl]-pyrrol als farbloses Öl.

$^1$H-NMR-Spektrum ($CDCl_3$, TMS intern):

$\delta$ = 2,65 (m, 2H); 3,25–3,6 (m, 1H); 4,23 (m, 2H); 6,28 (m, 1H); 6,68 (m, 5H); 7,59 (m, 1H); 10,0 (breit, 1H).

II. Herstellung von erfindungsgemässen Verbindungen

Beispiel 1

1,5 g 2-[2-(2,3-Epoxypropoxy)-phenyl]-pyrrol und 1 ml tert.-Butylamin werden in 5 ml Äthanol über Nach belassen und anschliessend das Lösungsmittel und überschüssiges Amin abdestilliert. Der wachsartige Rückstand wird in wenig Äthanol gelöst und tropfenweise mit ätherischer Fumarsäure versetzt. Das ausgefallene 2-[2-(3-tert.-Butylamino-2-hydroxypropoxy)-phenyl]-pyrrol-fumarat wird abgesaugt mit trockenem Äther gewaschen und getrocknet.

Ausbeute: 1,8 g (75% der Theorie) vom Schmp. 197 bis 198°C.

$C_{19}H_{26}O_4N_2$ (346)

| | | | |
|---|---|---|---|
| ber.: | 65,8 C | 7,6 H | 8,1 N |
| gef.: | 65,3 C | 7,5 H | 7,9 N |

Beispiel 2

1,5 g 2-[2-(2,3-Epoxypropoxy)-phenyl]-pyrrol und 3 ml Isopropylamin werden gemäss Beispiel 1 umgesetzt. Nach dem Umkristallisieren aus einem Methanol-Äthanol-Gemisch (1 : 1) werden 0,7 g (30% der Theorie) 2-[2-(3-Isopropylamino-2-hydroxypropoxy)-phenyl]-pyrrol-fumarat-hemihydrat vom Schmp. 176 bis 177°C erhalten.

$C_{18}H_{24}O_4N_2 \cdot ½\ H_2O$ (341)

| | | | |
|---|---|---|---|
| ber.: | 63,3 C | 7,3 H | 8,21 N |
| gef.: | 63,4 C | 7,2 H | 8,1 N |

Formulierungsbeispiele, die in üblicher Weise hergestellt werden:

1. Tabletten:

| | |
|---|---|
| a) Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

| | |
|---|---|
| b) Ein Wirkstoff der Formel I | 20 mg |
| Lactose | 178 mg |
| Avicel | 80 mg |
| Polywachs 6000 (Polyäthylenoxid) | 20 mg |
| Magnesiumstearat | 2 mg |
| | 300 mg |

| | |
|---|---|
| c) Ein Wirkstoff der Formel I | 50 mg |
| Polyvinylpyrrolidon (mittl. M.G. 25 000) | 170 mg |
| Polyäthylenglykol (mittl. M.G. 4000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10%iger wässriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4000), Hydroxypropylmethylcellulose, Talkum und Magnesiumstearat vermischt und zu Tabletten à 280 mg verpresst.

2. Beispiel für Dragees:

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8%igen wässrigen Lösung des Polyvinylpyrrolidons durch Sieb 1,5 mm granuliert, bei 50°C getrocknet und nochmals durch Sieb 1,5 mm gerieben. Das so erhaltene Granulat wird mit Magnesiumstearat gemischt und zu Drageekernen verpresst. Die erhaltenen Drageekerne werden in üblicher Weise mit einer Hülle überzogen, die im wesentlichen aus Zucker und Talkum besteht.

3. Kapselformulierung:

| | |
|---|---|
| Ein Wirkstoff der Formel I | 5,0 mg |
| Magnesiumstearat | 2,0 mg |
| Milchzucker | 19,3 mg |

4. Injektionslösung:

| | |
|---|---|
| Ein Wirkstoff der Formel I | 10 mg |
| Natriumchlorid | 9 mg |
| destilliertes Wasser, q.s. auf 1,0 ml | |

**Patentansprüche für die Vertragsstaaten: BE, CH, DE, FR, GB, IT, LU, NL, SE**

1. Verbindungen der allgemeinen Formel (I)

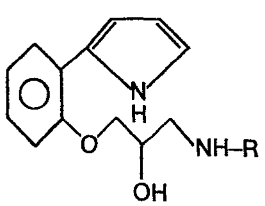
(I)

in der R einen am zum Stickstoffatom α-ständigen C-Atom verzweigten Alkylrest mit 3 bis 6 C-Atomen bedeutet und ihre physiologisch verträglichen Säureadditionssalze.

2. 2-[2-(3-tert.-Butylamino-2-hydroxy-propoxy)-phenyl]-pyrrol.

3. 2-[2-(3-Isopropylamino-2-hydroxy-propoxy)-phenyl]-pyrrol.

4. Verfahren zur Herstellung von Verbindungen der Formel I nach Anspruch 1, dadurch gekennzeichnet, dass man ein Pyrrol der allgemeinen Formel (II)

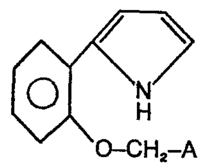
(II),

in der A den Rest $-CH-CH_2$ (O) oder $-CH(OH)-CH_2-B$, für eine nukleofuge Abgangsgruppe steht, bedeutet, mit einem Amin der allgemeinen Formel

$H_2N-R$,

in der R die für Formel I angegebenen Bedeutungen hat, zweckmässigerweise in einem Lösungsmittel und gegebenenfalls in Gegenwart eines säurebindenden Mittels bei Temperaturen von 50 bis 120°C umsetzt und die erhaltene Verbindung gegebenenfalls in ein physiologisch verträgliches Säureadditionssalz überführt.

5. Therapeutisches Mittel, gekennzeichnet durch einen Gehalt an einer Verbindung der allgemeinen Formel (I) nach Anspruch 1 oder ihres physiologisch verträglichen Säureadditionssalzes neben üblichen Trägerstoffen und Verdünnungsmitteln.

**Claims for the Contracting states BE, CH, DE, FR, GB, IT, LU, NL, SE**

1. A compound of the general formula (I)

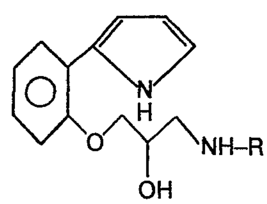
(I)

where R is alkyl of 3 to 6 carbon atoms which is branched at the carbon in the α-position to the nitrogen, and its physiologically acceptable acid addition salts.

2. 2-[2-(3-tert.-Butylamino-2-hydroxy-propoxy)-phenyl]-pyrrole.

3. 2-[2-(3-Isopropylamino-2-hydroxy-propoxy)-phenyl]-pyrrole.

4. A process for the preparation of a compound of the formula I as claimed in claim 1, characterized in that a pyrrole of the general formula (II)

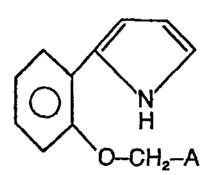
(II),

where A is $-CH-CH_2$ (O) or $-CH-CH_2-B$, B being a nucleofugic leaving group. is reacted with an amine of the general formula

$H_2N-R$

where R has the meanings given for formula I, advantageously in a solvent, and in the presence or absence of an acidbinding agent, at from 50 to 120°C, and the resulting compound may or may not be converted into a physiologically acceptable acid addition salt.

5. A therapeutic agent characterized by a content of a compound of the general formula (I) as claimed in claim 1, or of a physiologically acceptable acid addition salt thereof, in addition to conventional excipients and diluents.

**Revendications pour les Etats contractants: BE, CH, DE, FR, GB, IT, LU, NL, SE**

1. Composés de la formule générale (I)

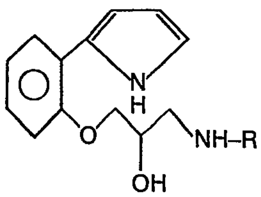

(I)

dans laquelle R désigne un radical alcoyle en $C_3$ à $C_6$ ramifié sur l'atome de carbone en position α par rapport à l'atome d'azote, ainsi que leurs sels d'addition d'acides physiologiquement acceptables.

2. Le 2-[2-(3-tertiobutylamino-2-hydroxy-propoxy)-phényl]-pyrrole.

3. Le 2-[2-(3-isopropylamino-2-hydroxy-propoxy)-phényl]-pyrrole.

4. Procédé de préparation de composés de la formule I suivant la revendication 1, caractérisé en ce que l'on fait réagir un pyrrole de la formule générale (II)

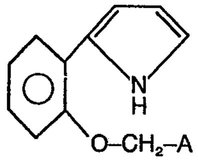

(II),

dans laquelle A représente un groupe 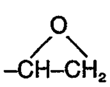 ou un groupe 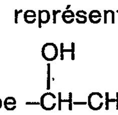, dans lequel B désigne un groupe de clivage nucléofuge, à des températures de 50 à 120°C, avantageusement dans un solvant et éventuellement en présence d'un fixateur d'acide, avec une amine de la formule générale

$$H_2N-R,$$

dans laquelle R possède les significations définies pour la formule I, le composé obtenu pouvant, le cas échéant, être transformé en un sel d'addition d'un acide physiologiquement acceptable.

5. Composition thérapeutique, caractérisée en ce qu'elle contient un composé de la formule générale (I) suivant la revendication 1 ou un sel d'addition d'un acide physiologiquement acceptable d'un sel composé à côté de véhicules et diluants habituels.

**Patentanspruch für den Vertragsstaat: AT**

Verfahren zur Herstellung von Verbindungen der Formel (I)

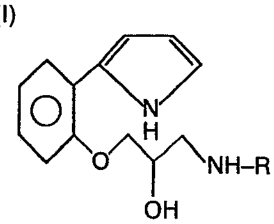

(I)

in der R einen am zum Stickstoffatom α-ständigen C-Atom verzweigten Alkylrest mit 3 bis 6 C-Atomen bedeutet und ihren physiologisch verträglichen Säureadditionssalze, dadurch gekennzeichnet, dass man ein Pyrrol der allgemeinen Formel (II)

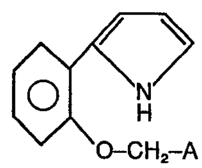

(II),

in der A den Rest 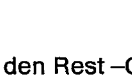 oder 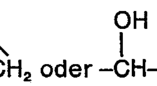, wobei B für eine nukleofuge Abgangsgruppe steht, bedeutet, mit einem Amin der allgemeinen Formel

$$H_2N-R,$$

in der R die für Formel I angegebenen Bedeutungen hat, zweckmässigerweise in einem Lösungsmittel und gegebenenfalls in Gegenwart eines säurebindenden Mittels bei Temperaturen von 50 bis 120°C umsetzt und die erhaltene Verbindung gegebenenfalls in ein physiologisch verträgliches Säureadditionssalz überführt.

**Claim for the Contracting state: AT**

A process for the preparation of a compound of the general formula (I)

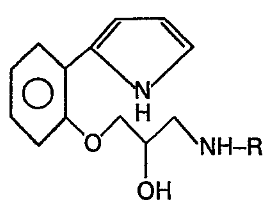

(I)

where R is alkyl of 3 to 6 carbon atoms which is branched at the carbon in the α-position to the nitrogen, and its physiologically acceptable acid addition salts, characterized in that a pyrrole of the general formula (II)

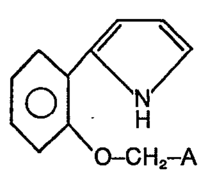

(II),

where A is 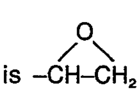 or $-CH-CH_2-B$, B being a

nucleofugic leaving group, is reacted with an tagwamine of the general formula

$$H_2N–R$$

where R has the meanings given for formula I, advantageously in a solvent, and in the presence or absence of an acid-binding agent, at from 50 to 120°C, and the resulting compound may or may not be converted into a physiologically acceptable acid addition salt.

**Revendication pour l'Etat contractant: AT**

Procédé de préparation de composés de la formule (I)

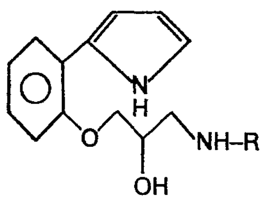

(I)

dans laquelle R désigne un radical alcoyle en $C_3$ à $C_6$ ramifié sur l'atome de carbone en position α par rapport à l'atome d'azote, ainsi que de leurs sels d'addition d'acides physiologiquement acceptables, caractérisé en ce que l'on fait réagir un pyrrole de la formule générale (II)

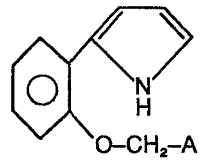

(II),

dans laquelle A représente un groupe 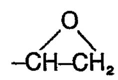 ou un groupe 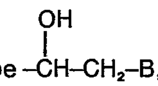, dans lequel B désigne un groupe de clivage nucléofuge, à des températures de 50 à 120°C, avantageusement dans un solvant et éventuellement en présence d'un fixateur d'acide, avec une amine de la formule générale

$$H_2N–R,$$

dans laquelle R possède les significations définies pour la formule I, le composé obtenu pouvant, le cas échéant, être transformé en un sel d'addition d'un acide physiologiquement acceptable.